# NEUE EUROPÄISCHE PATENTSCHRIFT

(11) **EP 1 512 694 B2**
(45) Veröffentlichungstag und Bekanntmachung des Hinweises auf die Entscheidung über den Einspruch: **03.11.2010**
(45) Hinweis auf die Patenterteilung: 13.09.2006
(21) Anmeldenummer: 04027711.3
(22) Anmeldetag: 27.11.2000
(51) Int. Cl.: C07K 14/435, A61K 39/35, G01N 33/68, A61P 37/08

(54) **Insektengift-Allergene mit verminderter IgE-Reaktivität und Verfahren zu ihrer Herstellung**
Wasp venom allergens with reduced IgE-reactivity
Allergènes des toxines de la guêpe ayant une réactivité IgE réduite

(30) Priorität: 01.12.1999 DE 19957904
(43) Veröffentlichungstag der Anmeldung: 09.03.2005
(62) Teilanmeldung aus: 00987296.1
(73) Patentinhaber: Merck Patent GmbH, 64293 Darmstadt (DE)
(72) Erfinder: Fiebig, Helmut, Prof. Dr. habil. nat., 21493 Schwarzenbek (DE); Suck, Roland, Dr., 22301 Hamburg (DE); Cromwell, Oliver, Dr., 23701 Suesel-Fassendorf (DE)

(56) Entgegenhaltungen:
- CA-A1- 2 393 975
- US-A- 5 804 201
- MONSALVE R.I. ET AL.: "Expressions of recombinant venom allergen, antigen 5 of yellowjacket (Vespula vulgaris) and paper wasp (Polistes annularis), in bacteria and yeast" PROTEIN EXPRESSION AND PURIFICATION, Bd. 16, August 1999 (1999-08), Seiten 410-416, XP002173771
- HOFFMAN DR & WOOD CL: "Allergens in Hymenoptera venom XI. Isolation of protein allergens from Vespula maculifrons (yellow jacket) venom" JOURNAL OF ALLERGY AND CLINICAL IMMUNOLOGY, Bd. 74, Nr. 1, 1984, Seiten 93-103, XP008040229
- SUCK R. ET AL.: "Purification and immunobiological characterization of folding variants of the recombinant major wasp allergen ves v 5 (antigen 5)" INTERNATIONAL ARCHIVES OF ALLERGY AND IMMUNOLOGY, Bd. 121, 1. April 2000 (2000-04-01), Seiten 284-291, XP001010205
- MARSTON F.: 'The purification of eukaryotic polypeptides synthesized in Escherichia coli' BIOCHEM J. Bd. 240, 01 Januar 1986, Seiten 1 - 12
- RUDOLPH R.; LILIE H.: 'In vitro folding of inclusion body proteins' THE FASEB JOURNAL Bd. 10, 01 Januar 1996, Seiten 49 - 56
- KING ET AL: 'Murine T and B Cell Responses to Natural and Recombinant Hornet Venom Allergen Dol m 5.02 and Its Recombinant Fragments' J.IMMUNOLOGY Bd. 154, 01 Januar 1994, Seiten 577 - 584
- GURR E.: 'Effect of Heat on the pH of Water and Aqueous Solutions' NATURE Bd. 195, 01 Januar 1962, Seiten 1199 - 1200
- HOFFMANN D.R.: 'Allergens in Hymenoptera venom XXV' J. ALLERGY CLIN. IMMUNOL Bd. 92, 01 Januar 1993, Seiten 707 - 716
- SAMBROK J. ET AL: 'Molecular Cloning - A Laboratory Manual - 2nd edition' COLD SPRING HARBOUR LABORATORY PRESS 01 Januar 1989, Seiten 1736 - 1744
- MARSTON F. ET AL: 'Ppurification of Calf Prochymosin (Prorewnnin) synthesized in Escherichia Coli' BIOTECHNOLOGY 01 September 1984, Seiten 800 - 804
- PAIN R. ET AL: 'Overview of Protein Folding' CURRENT PROTOCOLS IN PROTEIN SCIENCE 01 Januar 1995, Seiten 641 - 646

## Beschreibung

Die Erfindung betrifft rekombinante Wespengift Allergene Antigen 5, wobei sich besagte Allergene je nach Durchführung des Herstellungsverfahrens durch naturidentische oder naturfremde Faltungen (Konformationen) unterscheiden lassen.

Eine Anwendung für Faltungsformen, die dem natürlichen Molekül entsprechen, besteht in der Einzelallergen-differenzierten Diagnostik (*in vitro* oder *in vivo*) von Allergikern, speziell Insektengiftallergikern.

Die naturfremden Faltungsformen können als nebenwirkungsarme Therapeutika zur spezifischen Immuntherapie eingesetzt werden. Damit könnten diese rekombinanten Faltungsvarianten eine sicherere Behandlung als der Naturstoff bewirken. Das Verfahren ist so konzipiert, daß eine biotechnologische Herstellung unter Bedingungen, die für Pharmazeutika notwendig sind (GMP), durchgeführt werden kann.

Insektenstichallergien werden hauptsächlich von Wespen und Honigbienen verursacht und können zu schweren systemischen Symptomen bis hin zur potenziell tödlich verlaufenden Anaphylaxie führen (Müller, U.R., in: Insect sting allergy, Gustav Fischer Verlag; 1990). Bei den Typ 1-Allergie auslösenden Substanzen handelt es sich um Proteine, Glykoproteine oder Polypeptide des Insektenvenoms. Diese Allergene reagieren nach Injektion mit den bei sensibilisierten Personen an der Oberfläche von Mastzellen gebundenen IgE-Molekülen. Werden solche FcεRI-gebundenen IgE-Moleküle durch ein Allergen miteinander vernetzt, führt dies zur Ausschüttung von Mediatoren (z.B. Histamin, Leukotriene) und Zytokinen durch die Effektorzelle und damit zu den entsprechenden klinischen Symptomen.

Als allergene Bestandteile des Bienenvenoms wirken neben dem Peptid Melittin die Enzyme Hyaluronidase und Phospholipase A2 (Habermann, E, 1972, Science 177, 314-322). Im Fall der Wespe kommen als enzymatisch aktive Hauptallergene ebenfalls eine Hyaluronidase, die der des Bienenvenoms ähnlich ist (Hoffmann, D.R., 1986, J. Allergy Clin. Immunol. 78, 337-343) und eine Phospholipase A1 vor. Das wichtigste Hauptallergen des Wespengiftes ist das Antigen 5, für das bisher keine enzymatische Aktivität nachgewiesen werden konnte (King et al., 1978, Biochemistry 17, 5165-5174). Sämtliche der genannten Allergene sind bereits molekularbiologisch charakterisiert und die entsprechenden cDNA-Moleküle kloniert (u.a. Fang et al., 1988, PNAS, 895-899; Soldatova et al., 1993, FEBS, 145-149; Kuchler et al., 1989, Eur. J. Biochem. 184, 249-254). Mit Hilfe von cDNA Sequenzen ist es möglich, rekombinante Allergene herzustellen, die in der Diagnostik und Therapie von Allergien Verwendung finden könnten (Scheiner and Kraft, 1995, Allergy 50, 384-391).

Im Zusammenhang mit der vorliegenden Erfindung ist das Hauptallergen Antigen 5 von besonderer Bedeutung, da sich die Erfindung dieses Moleküls bedient. Es handelt sich dabei um ein ca. 25 kDa großes, nicht glykosyliertes Protein. Die Primärsequenz beinhaltet 8 Cysteinreste, was auf vier Disulfidbrücken hinweist (Hoffman, D.R., 1993, J. Allergy Clin Immunol. 92: 707-716). Ein klassischer Ansatz zur wirksamen therapeutischen Behandlung von Insektengiftallergien stellt die Spezifische Immuntherapie oder Hyposensibilisierung dar (Müller, U.R., in: Insect sting allergy, Gustav Fischer Verlag; 1990). Dabei werden dem Patienten natürliche Allergenextrakte in steigenden Dosen subkutan injiziert. Allerdings besteht bei dieser Methode die Gefahr von allergischen Reaktionen bis zum anaphylaktischen Schock. Da gerade im Fall von Insektenstichhyposensibilisierungen mit starken Reaktionen zu rechnen ist, wird zur Zeit ausschließlich stationär behandelt.

Eine Therapieoptimierung wäre mit rekombinant hergestellten Allergenen besonders bei der Insektenstichallergie möglich. Definierte, ggf. auf individuelle Sensibilisierungsmuster der Patienten abgestimmte Cocktails von hochreinen rekombinant hergestellten Allergenen (Scheiner and Kraft, 1995) könnten Extrakte aus natürlichen Allergenquellen ablösen. Realistische Perspektiven, die zu einer sicheren Hyposenibilisierung mit solchen rekombinanten Allergenen führen können, bieten gezielt mutierte rekombinante Allergene, bei denen IgE-Epitope spezifisch deletiert werden, ohne die für die Therapie essenziellen T-Zell Epitope zu beeinträchtigen (Schramm et al., 1999, J. Immunol. 162, 2406-2414).
Von der heterologen Expression in *E.coli* ist bekannt, daß die meisten eukaryontischen Proteine nicht oder nur in geringem Ausmaß die 'natürliche' Konformation annehmen. Eine Folge von diesen Fehlfaltungen ist häufig die Unlöslichkeit dieser Proteine. Dies ist besonders bei cysteinhaltigen Proteinen zu beobachten (Kuchler et al., 1989, Eur. J. Biochem. 184, 249-254). Insbesondere von Antigen 5 wurde berichtet, daß die Expression in Bakterien zu unlöslichen Aggregaten führt, die nicht die natürliche Konformation besitzen (Monsalve et al., 1999, Protein Express Purif. 16(3): 410-416). Solche unlöslichen Aggregate sind weder zur Diagnostik noch zur Therapie nutzbar.

Häufig werden die in *E.coli* unlöslichen Proteine für Forschungszwecke in einem eukaryontischen Expressionssystem dargestellt, wie z.B. Hefe oder Insektenzellen (Monsalve et al., 1999, Protein Express Purif 16(3): 410-416; Soldatova et al., 1998, J Allergy Clin Immunol 101:691-698). Nachteile der eukaryontischen Expressionssysteme stellen jedoch vor allem mögliche Hyperglykosylierungen (Grobe et al., 1999, Eur J Biochem 263:33-40), proteolytische Degradationsprozesse und vergleichsweise kleine Produktausbeuten dar (Glover and Hames (eds.), 1995, Expression Systems, IRL Press, Oxford-New York-Tokyo). Für die allergologische Anwendung im Sinne einer pharmazeutisch-medizinischen Diagnostik und Therapie sind solche Proteine deshalb meist ungeeignet.

Die Produkte des erfindungsgemäßen Verfahrens, welche die natürliche Konformation besitzen, können in der In-vitro- und In-vivo-Diagnostik von Wespenstichallergie, vorteilhaft angewendet werden. Diese naturidentische Faltungsform steht zur Detektion von IgE-Antikörpern in etablierten Verfahren zur Verfügung.

Andererseits können die mit Hilfe der Erfindung hergestellten Varianten, die sich durch eine im wesentlichen nicht oder nur partiell lge-reaktive Konformationen auszeichnen, als hypoallergene Komponenten in Präparaten zur spezifischen Immuntherapie angewendet werden. Unter dem Ausdruck "hypoallergen" wird oben und unten erfindungsgemäß eine verminderte bis fehlende, vorzugsweise um 5 bis 95 %, insbesondere 20 bis 85 %, verminderte Allergenität (gegenüber dem natürlichen Allergen) verstanden, welche durch eine verminderte IgE-Antwort bedingt ist.

Bei der vorliegenden Erfindung handelt es sich um eine Methode, mit der rekombinante Allergene in Bakterien (*E.coli*) hergestellt werden können. Durch hohe Anreicherung der unlöslichen Protein-Aggregate findet ein erster Reinigungsschritt statt. Diese Aggregate werden dann ohne Zusatz von Reduktionsmitteln denaturiert. In Abhängigkeit von den folgenden Dialysebedingungen werden unterschiedliche Faltungsformen erhalten. Entscheidend ist, daß es sich dabei um monomere und in destilliertem Wasser lösliche Moleküle handelt. Die eine lösliche Faltungsvariante besitzt eine dem natürlichen Allergen vergleichbare IgE-Reaktivität und kann demnach für diagnostische Zwecke verwendet werden. Eine solches Produkt wird durch Dialyse mit Cystein-haltiger Lösung erhalten.

Die anderen alternativen löslichen Faltungsvarianten sind strukturell verschieden von dem natürlichen Allergen und zeichnen sich durch verminderte oder fehlende IgE-Reaktivität aus. Aus diesem Grund sind solche Varianten geeignet, um eine verbesserte Immuntherapie zu ermöglichen. Ein solches hypoallergenes Produkt wird erfindungsgemäß durch Dialyse mit sauren Puffern, vorzugsweise in einem pH-Wert-Bereich zwischen 3,5 und 6,5, insbesondere zwischen 4,0 und 5,5 gewonnen.

Gegenstand der Erfindung ist somit rekombinantes Wespengiftallergen Antigen 5; welches dadurch gekennzeichnet ist, daß es eine verminderte IgE-Reaktivität, bzw. Allergenität besitzt. Erfindungsgemäß ist die Allergenität dieser Proteine bis zu 95%, im Vergleich zu dem natürlichen Allergen reduziert.

Gegenstand ist insbesondere ein entsprechendes rekombinantes Wespengiftallergen Antigen 5, insbesondere aus *Vespula vulgaris* und *Vespula germanica.*

Das rekombinant in Bakterien hergestellte, in destilliertem Wasser lösliche Wespengiftallergen Antigen 5 mit verminderter Allergenität bzw. IgE-Reaktivität kann durch Ausführen der folgenden Verfahrensschritte erhalten werden:
- Herstellen der allergenen Proteine in Bakterienzellen in unlöslicher Form als "inclusion bodies",
- Denaturieren der besagten unlöslichen Aggregate ohne Zusatz von Reduktionsmitteln,
- Überführen der denaturierten Produkte durch Dialyse unter Verwendung saurer Puffer in lösliche, monomere Allergene und
- Durchführen einer abschließende Dialyse gegen destilliertes Wasser und Isolieren des Antigens.

Dabei werden zur Dialyse saure Puffer, vorzugsweise Natriumacetat-Puffer mit einem pH-Wert zwischen 4.5 und 5,0, eingesetzt.

Gegenstand der Erfindung ist aber auch ein kombinanteres Wespengiftallergen Antigen 5 mit normaler Allergenität, bzw. IgE-Reaktivität, wobei zur Dialyse Cystein-haltige Lösungen anstelle saurer Puffer eingesetzt werden.

Gegenstand der Erfindung ist ferner eine pharmazeutische Zubereitung, welche ein entsprechendes rekombinantes Allergen mit verminderter, bzw. abgeschwächter IgE-Reaktivität sowie entsprechende Hilfs- und Trägerstoffe enthält.

Letztlich ist Gegenstand der Erfindung die Verwendung von Wespengiftallergene Antigen 5 mit normaler Allergenität bzw. IgE-Reaktivität, erhältlich nach einem entsprechenden oben oder unten beschriebenen Verfahren zur in vivo und in-vitro Diagnose von Insektenstichallergien.

### Im nachfolgenden wird das Verfahren im Detail beschrieben:

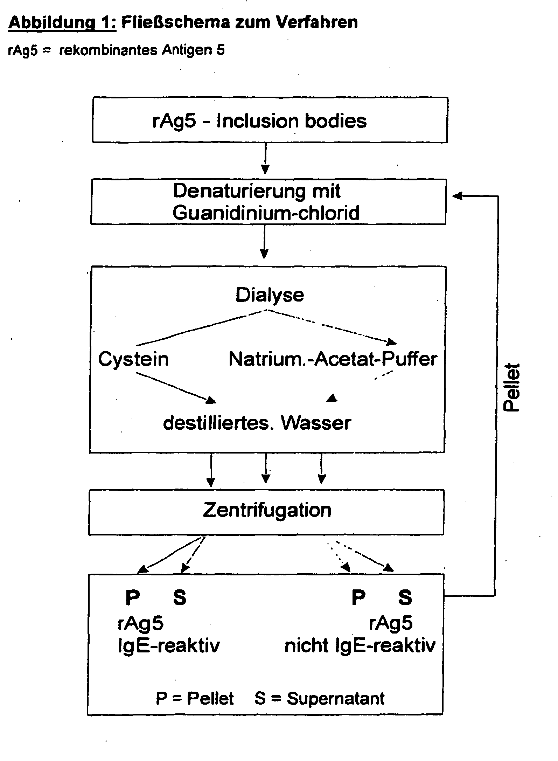

Beispielhaft wurden die Wespengiftallergene Antigen 5 von *Vespula vulgaris* (Ves v 5) und Antigen 5 von *Vespula germanica* (Ves g 5) in den Expressionsvektor pSE420 kloniert und in den K12-Bakterienstamm M15 pREP4 transformiert. Ein Fließschema des Verfahrens findet sich in Abbildung 1.

Zur Herstellung der rekominanten Allergene wird eine Vorkultur des Stammes zum Animpfen einer Expressionskultur genutzt. Die Expression, durch IPTG induziert, findet in einem Schikanekolben bei 37°C in LB-Medium und limitierter Sauerstoffversorgung statt (90 rpm/min). Die Bakterien werden nach 5 stündiger Expression durch Zentrifugation geerntet (5000xg, 10 min, 20°C). Der Bakterienaufschluß erfolgt nach Resuspension der Zellen in Puffer (50mM Tris/HCl, 25% (w/v) Sucrose, pH 8.0) durch Lysozymzugabe (10µg/g Naßgewicht). Es folgt die Zugabe des gleichen Volumens von Detergenzlösung (0.2 M NaCl, 1%(w/v) DOC, 1 %(w/v) Nonidet P40). Diese Aufschlußlösung wird anschließend mit Ultraschall behandelt (3 min auf Eis, 130 Watt, 0.5 s Impuls). Da die Expressionsprodukte primär als unlösliche Aggregate (Einschlusskörper, 'inclusion bodies') vorliegen, können sie aufgrund ihrer hohen Dichte von einem Großteil der übrigen Komponenten (Zellwandfragmente, Ribosomen etc.) durch Zentrifugation bei 3000 x g abgetrennt werden. Die weitere Reinigung erfolgt durch drei aufeinanderfolgende Waschschritte mit detergenzhaltigen Lösungen (1% Triton X-100). Im Anschluß werden die gereinigten Einschlusskörper durch Zugabe von Denaturierungspuffer (6M Guanidinium-Chlorid, 20 mM Tris/HCl, pH 8.0) aufgeschlossen und 2 h bei RT geschüttelt.

Zur Gewinnung von IgE-reaktiven Faltungsformen wird der Denaturierungsansatz in einen Dialyseschlauch (Ausschlussgrenze 12-14 kDa) gefüllt und gegen das 100 fache Volumen Cysteinlösung (5mM Cystein) 12 h unter Rühren bei RT dialysiert. Im Anschluß erfolgt eine Dialyse gegen destilliertes Wasser, um das Cystein zu entfernen.

Um Konformationen mit verminderter IgE-Reaktivität zu erhalten, wird die erste Dialyse gegen 20 mM Natriumacetat-Puffer (pH 5.0) durchgeführt werden. Auch hier erfolgt eine weiterer Dialyse gegen destilliertes Wasser. Nach Entnahme werden die wasserlöslichen Allergene durch Zentrifugation von den ausgefallenen Aggregaten abgetrennt. Der Überstand enthält die gewünschten löslichen rekombinanten Allergene. Anstelle von Natriumacetat-Puffer können auch andere saure Puffer verwendet werden, welche in einem Bereich von 3,5 bis 6,5, vorzugsweise 4,0 und 5,5 zu puffern vermögen. Beispiele solcher Puffersysteme sind in der Literatur hinlänglich beschrieben.

Die bei beiden Methoden anfallenden präzipitierten rekombinanten Allergene können nochmals denaturiert und nach dem gleichen Schema behandelt werden. Dadurch wird die Ausbeute deutlich erhöht.

Nach den Dialyseschritten sind die Produkte zu ca. 95% rein. Weitere Reinigungsschritte der basischen Insektengiftallergene sind Kationenaustauschchromatographie (Puffer pH 7.2) mit z.B. Source S (Pharmacia, Freiburg, Germany) und Gelfiltration. Die Gelfiltration dient neben der Abtrennung von hoch- und niedermolekularen Minimalverunreinigungen auch zur Entsalzung.

Die Qualitätskontrollen der Produkte basieren auf folgenden charakteristischen Eigenschaften, die tabellarisch für Antigen 5 zusammengestellt sind: nAntigen = natürliches Antigen

| **Eigenschaft** | **Faltung mit natürlicher IgE-Reaktivität** | **Faltung mit verminderter IgE-Reaktivität** |
|---|---|---|
| App. MW in der SDS-PAGE (nicht reduzierende Bed.) | 25 kDa | 26-27 kDa |
| Salzkonz. zur Elution in der Source S | 320 mM NaCl | 400 mM NaCl |
| Spaltung mit Protease V8 | 15 kDa Fragment + Peptide | Peptide < 10 kDa |
| Antigen 5 spezifische monoklonale Antikörper | Detektion durch 8E3, 1E11 | Detektion nur mit 8E3 möglich |
| Frequenz lgE-Reaktivität mit Allergikerseren | > 95% | <10% |
| Allergene Potenz | ähnlich nAntigen 5 | >10 x geringer als nAg5 |

Die verwendeten Aufreinigungstechniken sowie rekombinante Klonierungs und Expressionstechniken sind dem Fachmann bekannt und zugänglich und können durch bekannte ähnliche Verfahrenstechniken ersetzt werden.

## Patentansprüche

1. Rekombinant in Bakterien hergestelltes, in destilliertem Wasser lösliches Wespengiftallergen Antigen 5 mit verminderter Allergenität bzw. IgE-Reaktivität, erhältlich durch Ausführen der folgenden Verfahrensschritte:
- Herstellen der allergenen Proteine in Bakterienzellen in unlöslicher Form als "inclusion bodies",
- Denaturieren der besagten unlöslichen Aggregate ohne Zusatz von Reduktionsmitteln,
- Überführen der denaturierten Produkte durch Dialyse unter Verwendung saurer Puffer in lösliche, monomere Allergene und
- Durchführen einer abschließende Dialyse gegen destilliertes Wasser und Isolieren des Antigens.

2. Wespengiftallergen Antigen 5 gemäß Anspruch 1, **dadurch gekennzeichnet, daß** die Denaturierung mit Guanidinium-Chlorid erfolgt.

3. Wespengiftallergen Antigen 5 gemäß Anspruch 1 oder 2, **dadurch gekennzeichnet, daß** bei der Dialyse Puffer mit einem pH-Wert zwischen 3,5 und 6,5 eingesetzt werden.

4. Wespengiftallergen Antigen 5 gemäß einem oder mehreren der Ansprüche 1 bis 3, **dadurch gekennzeichnet, daß** es sich um ein Allergen der Spezies Vespula spec., insbesondere Vespula vulgaris und Vespula germanica und Paravespula spec. handelt.

5. Pharmazeutische Zubereitung, enthaltend ein Wespengiftallergen Antigen 5 gemäß einem oder mehreren der Ansprüche 1 bis 4 sowie entsprechende Hilfs- und Trägerstoffe.

6. Verwendung von Wespengiftallergen Antigen 5 gemäß einem oder mehreren der Ansprüche 1 bis 4 zur Herstellung eines Arzneimittels zur spezifischen lmmuntherapie bzw. Hyposensibilisierung von Wespengiftallergikern.

7. Rekombinant in Bakterien hergestelltes, in destilliertem Wasser lösliches Wespengiftallergen Antigen 5 mit normaler Allergenität bzw. IgE-Reaktivität, erhältlich durch Ausführen der folgenden Verfahrensschritte:
- Herstellen der allergenen Proteine in Bakterienzellen in unlöslicher Form als "inclusion bodies",
- Denaturieren der besagten unlöslichen Aggregate ohne Zusatz von Reduktionsmitteln,
- Überführen der denaturierten Produkte durch Dialyse unter Verwendung cystein-haltiger Lösungen in lösliche, monomere Allergene und
- Durchführen einer abschließende Dialyse gegen destilliertes Wasser und Isolieren des Antigens.

8. Wespengiftallergen Antigen 5 gemäß Anspruch 7, **dadurch gekennzeichnet, daß** es sich um ein Allergen der Spezies Vespula spec., insbesondere Vespula vulgaris und Vespula germanica und Paravespula spec. handelt.

9. Verwendung von Wespengiftallergen Antigen 5 gemäß einem oder mehreren der Ansprüche 7 oder 8, zur *in-vitro* Diagnose von Wespenstichallergien.

## Claims

1. Wasp venom allergen antigen 5 having reduced allergeneity or IgE reactivity, prepared recombinantly in bacteria, soluble in distilled water, obtainable by carrying out the following process steps:
- preparation of the allergenic proteins in bacterial cells in insoluble form as inclusion bodies,
- denaturing of the said insoluble aggregates without addition of reducing agents,
- conversion of the denatured products into soluble, monomeric allergens by dialysis using acidic buffers and
- performance of a final dialysis against distilled water and isolation of the antigen.

2. Wasp venom allergen antigen 5 according to Claim 1, **characterised in that** the denaturing is carried out using guanidinium chloride.

3. Wasp venom allergen antigen 5 according to Claim 1 or 2, **characterised in that** buffers having a pH of between 3.5 and 6.5 are employed in the dialysis.

4. Wasp venom allergen antigen 5 according to one or more of Claims 1 to 3, **characterised in that** it is an allergen of the species Vespula spec., in particular Vespula vulgaris and Vespula germanica, and Paravespula spec.

5. Pharmaceutical preparation comprising a wasp venom allergen antigen 5 according to one or more of Claims 1 to 4 and corresponding adjuvants and excipients.

6. Use of wasp venom allergen antigen 5 according to one or more of Claims 1 to 4 for the preparation of a medicament for the specific immunotherapy or hyposensitisation of wasp venom allergy sufferers.

7. Wasp venom allergen antigen 5 having normal allergeneity or IgE reactivity, prepared recombinantly in bacteria, soluble in distilled water, obtainable by carrying out the following process steps:
- preparation of the allergenic proteins in bacterial cells in insoluble form as inclusion bodies,
- denaturing of the said insoluble aggregates without addition of reducing agents,
- conversion of the denatured products into soluble, monomeric allergens by dialysis using cysteine-containing solutions and
- performance of a final dialysis against distilled water and isolation of the antigen.

8. Wasp venom allergen antigen 5 according to Claim 7, **characterised in that** it is an allergen of the species Vespula spec., in particular Vespula vulgaris and Vespula germanica, and Paravespula spec.

9. Use of wasp venom allergen antigen 5 according to one or more of Claims 7 or 8 for the in vitro diagnosis of wasp sting allergies.

## Revendications

1. Antigène 5 allergène de venin de guêpe présentant une allergénicité réduite ou une réactivité des IgE réduite, préparé de façon recombinante dans des bactéries, soluble dans de l'eau distillée, pouvant être obtenu en mettant en oeuvre les étapes de processus qui suivent :
- préparation des protéines allergéniques dans des cellules bactériennes sous une forme insoluble en tant que corps d'inclusion,
- dénaturation desdits agrégats insolubles sans ajout d'agent réducteur,
- conversion des produits dénaturés selon des allergènes monomériques solubles par dialyse en utilisant des tampons acides et
- réalisation d'une dialyse finale vis-à-vis d'eau distillée et isolation de l'antigène.

2. Antigène 5 allergène de venin de guêpe selon la revendication 1, **caractérisé en ce que** la dénaturation est mise en oeuvre en utilisant du chlorure de guanidinium.

3. Antigène 5 allergène de venin de guêpe selon la revendication 1 ou 2, **caractérisé en ce que** des tampons présentant un pH entre 3,5 et 6,5 sont utilisés au niveau de la dialyse.

4. Antigène 5 allergène de venin de guêpe selon une ou plusieurs des revendications 1 à 3, **caractérisé en ce qu'**il s'agit d'un allergène des espèces Vespula spec., en particulier Vespula vulgaris et Vespula germanica, et Paravespula spec.

5. Préparation pharmaceutique comprenant un antigène 5 allergène de venin de guêpe selon une ou plusieurs des revendications 1 à 4 et des adjuvants et excipients correspondants.

6. Utilisation d'un antigène 5 allergène de venin de guêpe selon une ou plusieurs des revendications 1 à 4 pour la préparation d'un médicament pour l'immunothérapie spécifique ou l'hyposensibilisation spécifique de personnes qui souffrent d'une allergie au venin de guêpe.

7. Antigène 5 allergène de venin de guêpe présentant une allergénicité normale ou une réactivité des IgE normale, préparé de façon recombinante dans des bactéries, soluble dans de l'eau distillée, pouvant être obtenu en mettant en oeuvre les étapes de processus qui suivent :
- préparation des protéines allergéniques dans des cellules bactériennes sous une forme insoluble en tant que corps d'inclusion,
- dénaturation desdits agrégats insolubles sans ajout d'agent réducteur,
- conversion des produits dénaturés selon des allergènes monomériques solubles par dialyse en utilisant des solutions contenant de la cystéine et
- réalisation d'une dialyse finale vis-à-vis d'eau distillée et isolation de l'antigène.

8. Antigène 5 allergène de venin de guêpe selon la revendication 7, **caractérisé en ce qu'**il s'agit d'un allergène des espèces Vespula spec., en particulier Vespula vulgaris et Vespula germanica, et Paravespula spec.

9. Utilisation d'un antigène 5 allergène de venin de guêpe selon une ou plusieurs des revendications 7 ou 8 pour le diagnostic in vitro d'allergies au dard de guêpe.
